# EUROPEAN PATENT APPLICATION

(11) **EP 2 939 622 A1**
(43) Date of publication of application: **04.11.2015**
(21) Application number: 15165836.6
(22) Date of filing: 30.04.2015
(51) Int. Cl.: A61B 17/70

(54) **SYSTEM FOR CORRECTION OF THE SPINE CURVATURES**

(30) Priority: 30.04.2014 US 201461986561 P
(71) Applicant: Sanpera Trigueros, Ignacio, 07015 Palma de Mallorca (ES); Burgos Flores, Jesús, 28042 Madrid (ES); Hevia Sierra, Eduardo, 28033 Madrid (ES)
(72) Inventor: Sanpera Trigueros, Ignacio, 07015 Palma de Mallorca (ES); Burgos Flores, Jesús, 28042 Madrid (ES); Hevia Sierra, Eduardo, 28033 Madrid (ES)
(74) Representative: Iglesias Monravá, José Mª

(57) **Abstract**

System comprising for each vertebra at least one bone anchoring element (10) having an axial part (11) to be anchored into a vertebra (V) and a head (12) pivotally connected to a receiving part (21) which is attached to a hollow threaded elongate member (22) which include two longitudinal slots (24) on opposition allowing insertion there through of a corrective rod (30), being displaceable by means of a threaded pusher; wherein the threads (41) of the threaded pusher (40) have a zip configuration with protrusions (46), comprising at least a thread distal portion protruding relative to a thread more proximal portion, said zip configuration being complementary with the threads (25) of the threaded elongated member (22).

## Description

### Field of the invention

The present invention provides a simplified spine curvatures correction system for reducing spinal deformities including without limitation scoliosis, using at least a bone anchoring element, such as pedicle screw, placed on each of the spinal vertebrae of the spine to be corrected, in combination with alignment elongated members or extenders pivotally connected in a single plane direction to said anchoring element and associated with corrective rods engaged with said extenders, running along a transverse plan, said corrective rods providing implant rods.

The spinal column is a complex system of bones and connective tissues that provides support for the human body and protection for the spinal cord and nerves. The adult spine is comprised of 33 vertebral bodies but the sacral and coccyx are fused so providing in fact 24 vertebral bodies, which are subdivided into three areas including seven cervical vertebrae, 12 thoracic vertebrae and five lumbar vertebrae. Between each vertebral body is an intervertebral disc that cushions and dampens the various translational and rotational forces exerted on the spinal column.

There are various disorders, diseases and types of injury which the spinal column may experience in a lifetime. The problems may include but are not limited to scoliosis, kyphosis, excessive lordosis, spondylolisthesis, slipped or ruptured discs, degenerative disc disease, vertebral body fracture, and tumors. Persons suffering from any of the above conditions typically experience extreme or debilitating pain and often times diminished nerve function.

### Background of the invention

One of the more common solutions to any of the above mentioned conditions involves a surgical procedure known as spinal fusion. A spinal fusion procedure involves fusing two or more vertebral bodies in order to eliminate motion at the intervertebral disc or joint. To achieve this, natural or artificial bone are placed between the vertebrae and frequently a hardware is used to instrument the involved vertebrae to avoid movement and in this way facilitating fusion. In this way damaged or diseased vertebrae are connected to healthy adjacent vertebrae to stabilize the spine while the bone grows and fusion takes place.

The mechanical hardware used to immobilize the spinal column typically involves a series of bone screws and metal rods or plates. When the spine surgery is posteriorly performed, one of the common practices is to place bone screws into the vertebral bodies and then connect a metal rod (usually of titanium or chromium-cobalt alloy or steel) between the bone screws thus creating a rigid structure between adjacent vertebral bodies.

Current systems perform a segmental correction of the curvatures of the spine, immobilizing a group of vertebrae of the spinal deformity center, regardless of the fact that as it is well known scoliosis deformity is a rotating deformity with maximum rotational deformity at the central vertebra of the curve and this deformity decreasing progressively towards the curve ends (periphery), so that the correction required in each vertebrae included in the curve deformity is different and various corrective forces are needed at each level.

Several approaches in the field are following detailed:
A spinal stabilization system suitable for performing spine surgery appears disclosed in EP 2366349, US 7563264 and US 7491218, where a bone fastener assembly with a collar that allows for angulation of a bone fastener relative to the collar in a conical range of motion is revealed. US7914558 and US7691132 disclose a similar method for inserting a spinal stabilization system in a human spine.
US 8147524 refer to a method of reducing a spinal deformity where a pair of bone anchors is attached of each of the vertebrae of the spinal section injured and the pair of bone anchor are interconnected with a bridge member to which correction metal rods as previously referred are attached.
US 2011/0319938 disclose a coplanar deformity correction system involving a bone anchor assembly including a bone anchor, a receiver mounted to the bone anchor, a saddle within the receiver, a spacer within the receiver and an engaging member.
US 8221474 reveals a method for assembling a system for correcting alignment of a spinal column of a patient using a des-rotation handle to a transverse bridge between first and second implant holders attached to a vertebra.
FR 2971698 disclose a device for the correction of spine deformations comprising pedicle screws arranged to be connected by a rod bent implantable, rods intended to be engaged through different tubular elements to align corresponding to the correction of said spine, by translation, rotation and rocking of the vertebrae.
US 8221426 disclose a spinal alignment system comprising a plurality of pedicle assemblies including pedicle posts and adapter to couple the pedicle posts and a holder to couple to the adapter.

The system of this invention applies the mechanical correction that specifically requires each vertebra.

EP 1774919 refers to a bone anchoring device revealing anchoring elements for a spinal alignment system wherein the movement of the anchoring element before locking is limited to a single plane by a form-fit connection between a head of the anchoring element an a pressure element.

EP 2373238 disclose a device for the spine deformations correction comprising a pedicle screw arranged to be connected by a rod bent implantable, a tubular hollow element threaded on its outer surface including two longitudinal slots on opposition allowing insertion there through of the corrective rod, being said tubular hollow element removable connected to the pedicle screw, and being said rod implantable by means of an axially guided pusher, guided and partially inserted in said slots and pushed by a cylindrical hollow threaded pusher, threaded into said hollow element.

In this solution, the hollow tubular member slots are not open at the tubular member distal end, because there is a hinge mechanism for opening and removing the tubular element from the pedicle screw, thus the rod cannot be inserted into the slots axially from above, hampering its installation.

Cited current systems, used for the spinal deformities correction, includes implantable pedicle screws and removable extenders thereby improving the lever arm to apply corrective forces on each vertebra, and an implantable rod, but said systems have a complex constitution, due to space limitations, the required forces to be applied on the vertebrae and the need to separate implantable parts from removable parts.

The present invention proposed system provides an extremely simple, and compact assembly, able to apply the forces required during the rod implantation in the spinal column.

### Summary of the invention

The present invention proposes a system for correction of the spine curvatures, comprising for each of a plurality of vertebrae of a section of the spine to be corrected:
- at least one bone anchoring element having an axial part configured to be anchored into a vertebra and a head;
- said head of said bone anchoring element having connected or pivotally connected a receiving part to which a threaded elongate member, extending along a longitudinal axis, is attached defining a housing space in a head proximal position;
- said threaded elongated member being a tubular hollow element, and including two longitudinal slots on opposition, allowing insertion there through in a plurality of said elongated members of a corrective rod, said longitudinal slots allowing said corrective rod to be displaced, by means of a threaded pusher, from the insertion position, in the distal part of the longitudinal slots, to the implanted position, in the proximal part of the longitudinal slots, reaching said housing to become there implanted reducing the spinal deformity;
- said threaded elongate member is at least partially removable once corrective rod is implanted.

Said bone anchoring element, typically a pedicle screw, has a receiving part connected, or pivotally connected to said head, by means of an articulation, to provide a movement of the threaded elongated member, or extenders, respect said anchoring element in a single plane, allowing the movements needed to avoiding the transmission of certain mechanical stresses to the vertebra that may be at risk during the spine correction, and at the same time providing a solid anchoring point on each vertebra in which a correction force can be applied for the correction of the curvature.

The single plane movement can provide only a cephalic-caudal mobility (allowing movement in the sagittal axis) or provide a lateral-medial mobility (transverse axis).

Said connection can be also a non-articulated connection, or a free-articulated connection, allowing movement in any articulated direction.

The slots guide the rod from the a distal position on relation with the screw head, where the rod is placed in the initial insertion position, prior its implantation and prior spine curvature correction, to a proximal position in which the bar is seated in the housing in the implantation position, resulting in this shift correction forces on the vertebrae, and producing the correction.

The housing space, defined by the receiving part and a proximal portion of the elongated member, is the space where the rod has to be placed to be implanted, and has a rod complementary shape.

The threaded elongated member connected to said anchoring element can be used as a lever arm to apply said correction force on said vertebra through said receiving part.

The invention proposed a system different from the state of art, in which:
- said threaded elongated member and the receiving part are integrated into the same element;
- said threaded elongated member is cylindrical, and is threaded on its inner surface;
- said slots extend from the threaded elongated member proximal end to the threaded elongated member distal end, completely dividing the threaded elongated member into two halves;
- said threaded pusher is inserted and threaded into said threaded elongated member, and comprises a driving head;
- at least some threads of the threaded pusher has a zip configuration with protrusions, comprising at least a threaded distal portion protruding relative to a more threaded proximal portion, said zip configuration is complementary with the threads of the threaded elongated member.

The threaded elongated member is hollow and has two opposite longitudinal slots, which completely divide the threaded elongated member in two halves, said two halves being connected only through the receiving part, and allowing a rod to be axially inserted in the elongated member axial direction into said slots, through the distal end of the threaded elongated member. A single rod can be simultaneously inserted in a plurality of elongated members slots, anchored to a plurality of vertebrae.

After the insertion of the rod into the slots, the threaded pusher can be threaded into the threaded inner surface of each threaded elongated member.

Screwing the threaded pusher into the threaded elongated member, using a screwdriver or similar tool coupled to the driving head of said threaded pusher, it push the rod along the slots, producing the forces needed to correct the spine curvature, and transmitting said forces to the vertebra by the anchoring element.

Because the threaded pusher is located within the threaded elongate member, the axial force produced by screwing said threaded pusher against the rod produce also a radial force against the threaded elongated member, and because the threaded elongated member is completely divided, this radial force produce the opening of said threaded elongated member, and the consequent uncoupling of the thread.

To prevent this uncoupling, protrusions are provided on at least some threads of the threaded pusher, wherein the thread has a distal and a proximal portion, and wherein said distal thread portion protrudes relative to a more thread proximal part of the same thread. These threads with protrusions, in combination with complementary threads of the threaded elongated member, produce a zip configuration, which avoid the opening of the threaded elongated member and the threads uncoupling due said radial force.

This solution permits a very simple rod insertion into the slots due the total division of the threaded elongated member, and a very simple screwing operation to place the rod on its implanted position due the threaded pusher with zip configuration, using only few steps and a minimal number of devices, simplifying therefore the actually existing systems.

The threaded elongated member has a weakening zone near or coincident with its proximal end. The weakened zone allows easily removing at least a portion of the threaded elongated member, by breakage or cutting through the weakened area. Due the division of the threaded elongated member in two halves, is easy to bend and to produce the breakage of each halve separately.

This weakening zone can be, for example, an annular groove or an indentation in the tubular threaded elongated member.

The removable part of the threaded elongated member is the non-implantable portion and the remaining portion is the implantable portion which includes said receiving part and surrounds said housing.

The threaded pusher has also a weakened area, which defines an implantable portion, and a non-implantable removable portion.

The threaded pusher weakened area can be, for example, two opposite parallel lateral recesses.

### Brief description of the drawings

Fig. 1 illustrate a schematic view of an example of the anchoring element, in the form of pedicle screw, with the corresponding receiving portion and threaded elongate member, said receiving portions being pivotable in a single plane, for correction of the spine deformation, and showing also the threaded pusher, and a rod (showed in transverse section) in a uncoupled position;
Fig. 2 illustrate the same schematic view of Fig. 1, with a rod inserted into the slots in a insertion position, and being the threaded pusher coupled into the threaded elongated member distal end;
Fig. 3 illustrate the same schematic view of Fig. 2, with the rod seated into the housing, set by the threaded pusher inserted to its most proximal position;
Fig. 4 illustrate the same schematic view of Fig. 3, with one non-implantable portion of the threated elongated member partially removed, and a schematic view of a breaking tool able to remove the non-implantable portion of the threated elongated member is also illustrate;
Fig. 5 illustrate the same schematic view of Fig. 4, with both non-implantable portions of the threaded elongated member being removed;
Fig. 6 illustrate the same schematic view of Fig. 5, with all the non-implantable portion of the threaded pusher being removed, remaining only the implantable portion;

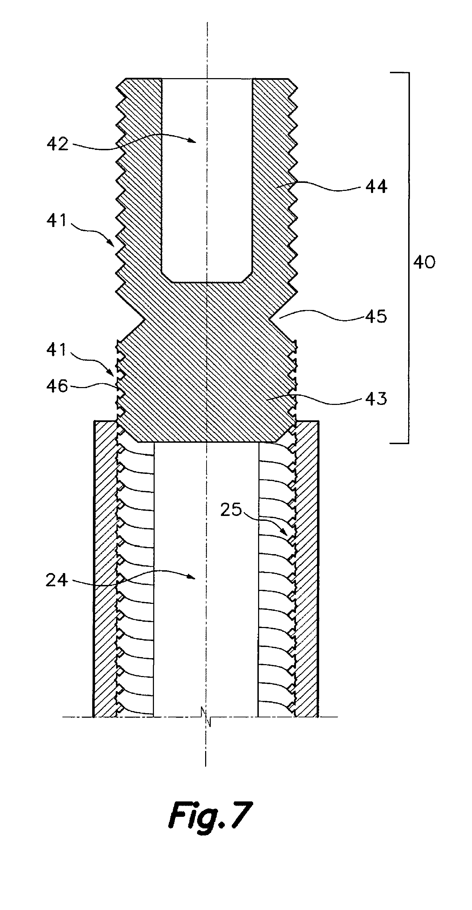

Fig. 7 illustrate a detail view of the threaded pusher in a transverse section, and the threaded elongated member distal end, with its thread with protrusions, according to a first embodiment;
Fig. 8 illustrate a detail partial view of the threaded pusher in a transverse section, with its thread with protrusions, according the embodiment shown in Fig. 7;
Fig. 9 illustrate a detail partial view of the threaded pusher in a transverse section, with its thread with protrusions, according to an alternative embodiment of zip configuration;
Fig. 10 illustrate a schematic view of a vertebra with two anchoring elements anchored, having one anchoring element an implantable rod in the implanted position, and only the implantable portion of the threaded elongated member and pusher.

### Detailed description of the drawings

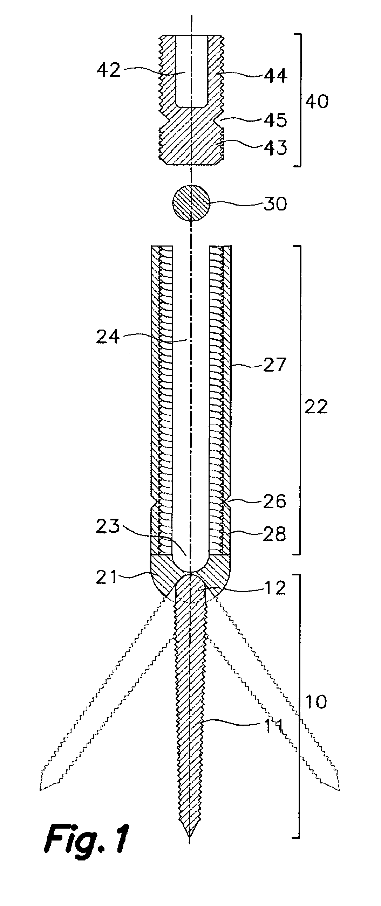

Fig. 1 illustrates the principle of this invention, wherein a system is provided to correct a spine curvature, including:
- an anchoring element 10;
- a receiving part 21 connected to said anchoring element 10;
- a threaded elongated member 22;
- an implantable rod 30;
- a threaded pusher 40.

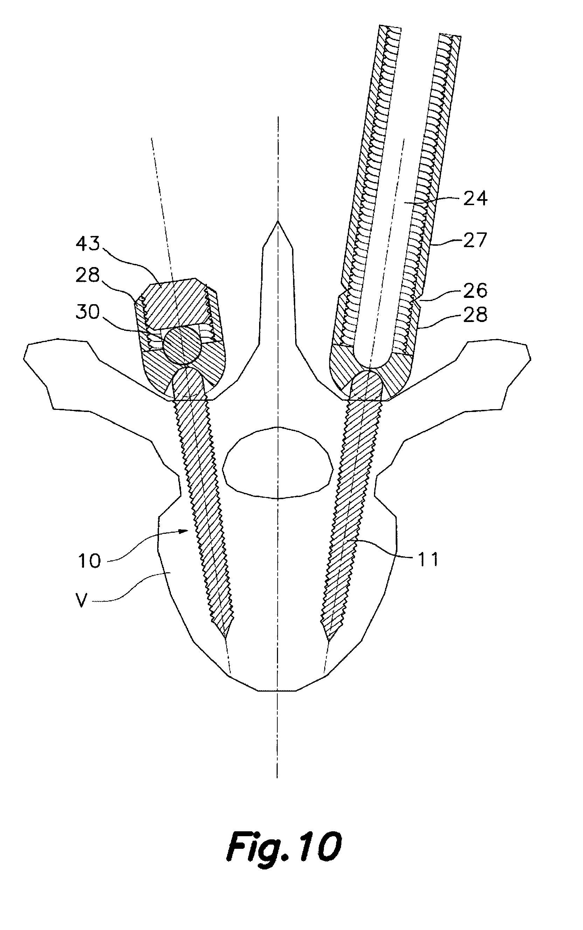

The anchoring element 10 is, according to a non-limiting embodiment example, a pedicle screw and comprises a threaded shaft as a axial part 11, configured to be anchored into a vertebra V, and a head 12, as shown in Fig. 10.

Said head 12 has two faces which are located opposite to each other and which have planar outer surface and confronted to said two opposite planar surfaces of the head two directly opposed shaped surfaces are defined in a receiving part 21 embedded in the threaded elongated member 22 proximal end, conforming a single element. Due this connection with said head 12, in this embodiment, the receiving part 21 can pivot in a single plane direction relative to the anchoring element 10. This pivot direction can be a sagittal direction or a transversal direction, allowing a kyphosis or lordosis correction or allowing a concavity or convexity correction.

Optionally two anchoring elements 10 can be attached to the same vertebra V, having both anchoring elements 10 different pivot direction, and in combination being able to correct any type of curvature.

As shown on Fig. 1, 2, 3 and 4, the threaded elongated member 22 is a hollow cylindrical tube anchored by one end to the receiving part 21, threaded on its inner surface, and totally divided in two halves by two longitudinal opposed and parallel slots 24 on the peripheral wall. Both halves stay anchored and connected to the receiving part 21.

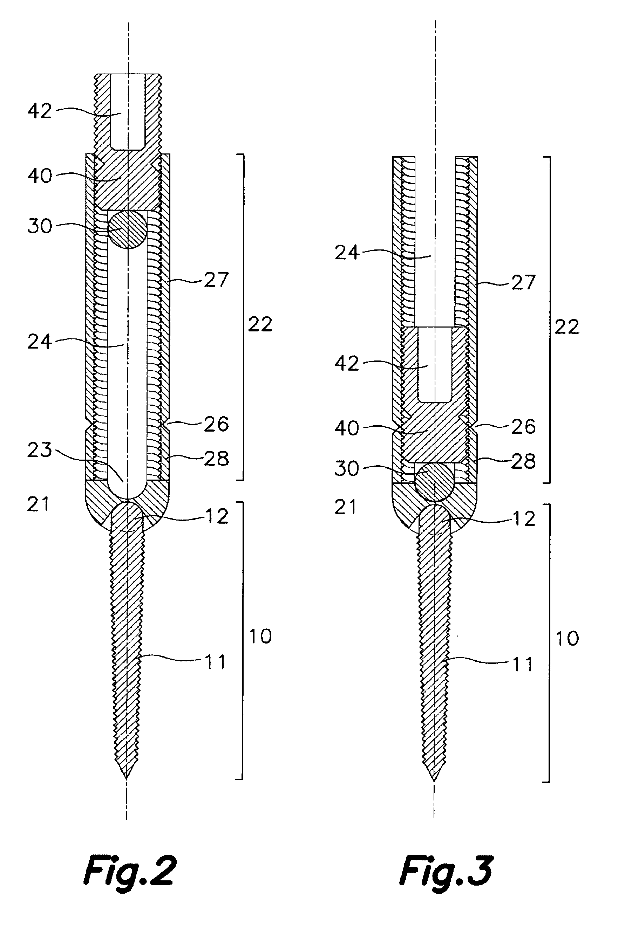

Said slots 24 permit the insertion there through of an implantable rod 30 into the threaded elongated member 22, positioning the rod 30 in the insertion position, in the threaded elongated member 22 distal portion (Fig. 2).

Once the implantable rod 30 is in the insertion position, the threaded pusher 40 is inserted and threaded into the hollow threaded elongated member 22, using a screwdriver or similar tool (not shown), coupled to the driving head 42 of the threaded pusher 40.

Screwing said threaded pusher 40, the rod 30 is pushed down along the slots 24. Being the rod 30 simultaneously inserted through slots 24 of a plurality of threaded elongated members 22, connected to anchoring elements 10, anchored to a plurality of consecutive vertebrae V, this displacement of the rod 30 through the slots 24 produce correction forces on said vertebrae V, producing its displacement and its correction, due the corrective shape preformed on the rod 30 previous its implantation. Being preferably all the anchoring elements 10 connections, a single plane pivotal connection, being all these single planes parallel to each other.

The threaded pusher 40 pushes the rod 30 until it reaches the implantation position illustrated on Fig. 3, in the housing space 23 defined and surrounded by receiving part 21 and threaded elongated member proximal end.

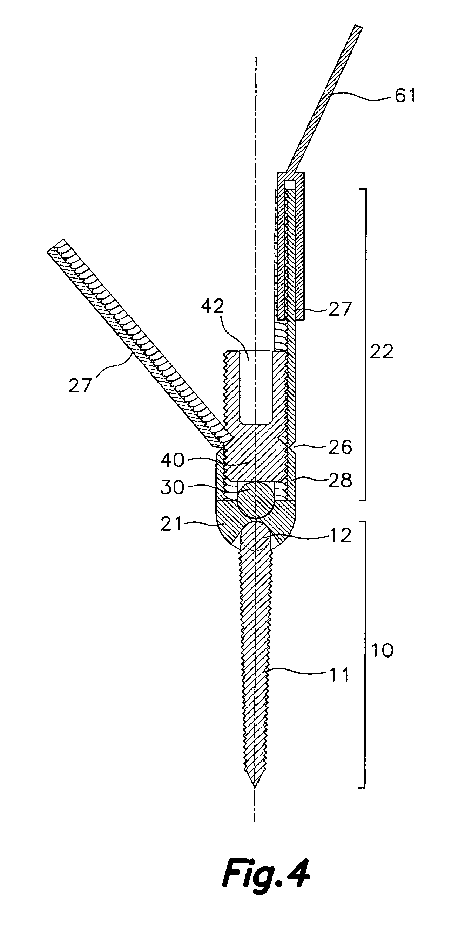

Once the rod 30 is placed in the implanted position, a non-implantable portion 27 of the threaded elongated member 22 and a non-implantable portion 44 of the threaded pusher 40 are removed, using a breaking tool 61 (Fig. 4), being the remaining portions the threaded elongated member 22 implantable portion 28, and the threaded pusher 40 implantable portion 43, which can be implanted with the rod on the spine.

The threaded pusher 40 implantable portion 43 stays threaded into the threaded elongated member 22 implantable portion 28, retaining the rod 30 in the implantable position (Fig. 10).

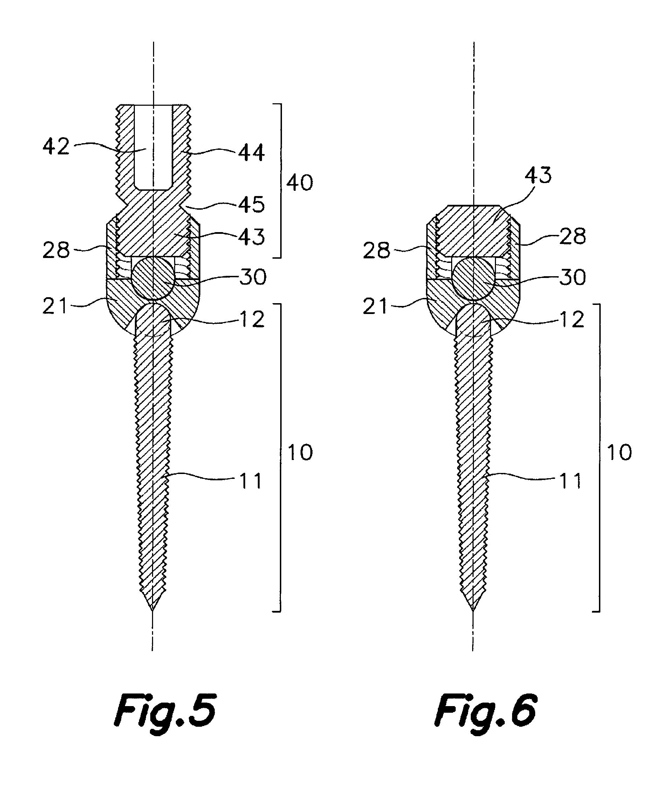

The division between the implantable 28 and 43 and the non-implantable portions 27 and 44 is defined by a weakening zone 26 of the threaded elongated member 22, one on each halve, and by a weakening zone 45 of the threaded pusher 40. Said weakening zones 26 and 45, illustrated in Fig. 1 to 6, are on this embodiment transversal grooves which facilitate the bending and breakage, or cutting of said threaded elongated member 22 and said threaded pusher 40, using for example the breaking tool 61 of Fig. 4.

Screwing the threaded pusher 40 into the threaded elongated member 22 against the rod 30 produces longitudinal forces and also radial forces in radial directions. These radial forces tend to cause separation of the two threaded elongated member 22 halves, producing its opening and the threaded pusher 40 uncoupling of the threaded elongated member 22. To prevent this uncoupling, some threads 41 of the threaded pusher 40 are equipped with a protrusion 46, protruding a pusher thread distal portion with respect to a proximal portion of the same thread. These protrusions 46, in combination with a complementary elongated member threads, produces a zip configuration, being impossible uncoupling both threaded parts in a radial direction.

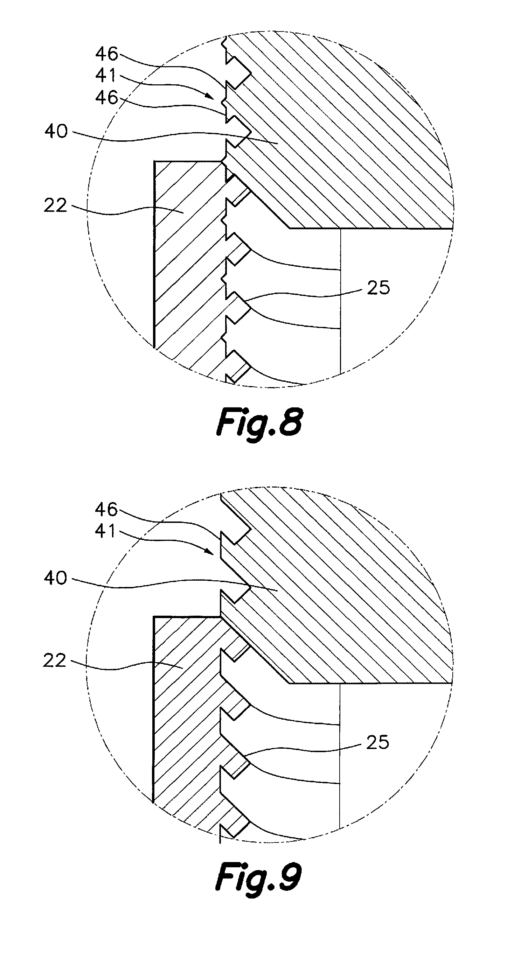

Examples of said protrusions are shown in Fig. 8 and 9, which shows how a single thread 41 has a thick distal portion, a thinner medium portion, and thick proximal portion, protruding said distal portion relative to the medium portion (medium portion is more proximal than the distal portion). Every thread 41 can be symmetrical, having same protrusions 46 on both sides, or can have only one protrusion 46 on one side of the thread 41.

This zip configuration will prevent the bending and breakage of the threaded elongated members 22, while the threaded pusher 40 being coupled on it, but it is also necessary to remove the non-implantable portion 27 of the threaded elongated member 22, keeping the threaded pusher 40 retaining the rod 30 on the implantation position. To achieve this, only the threads 41 of the thread pusher 40 implantable portion 43 has said protrusions 46, thus being the rod 30 retained in the implantable position by the threaded pusher 40, the threaded pusher 40 implantable portion 43 will be screwed into the threaded elongated member 22 implantable portion 28. The threaded pusher 40 non-implantable portion 44 will have normal threads 41, or any threads 41 allowing the uncoupling and the bending and breakage of the threaded elongated member 22 non-implantable portion 27.

## Claims

1. System for correction of the spine curvatures, comprising for each of a plurality of vertebrae of a section of the spine to be corrected:
• at least one bone anchoring element (10) having an axial part (11) configured to be anchored into a vertebra (V) and a head (12);
• said head (12) of said bone anchoring element (10) having connected, or pivotally connected, a receiving part (21) to which a threaded elongate member (22) extending along a longitudinal axis is attached, defining a housing (23) space in a head (12) proximal position;
• an implantable rod, preformed with a corrective curvature;
• said threaded elongated member (22) being a tubular hollow element, and including two longitudinal slots (24) on opposition, allowing insertion there through in a plurality of said elongated members (22) of a corrective rod (30), said longitudinal slots (24) allowing said corrective rod (30) to be displaced, by means of a threaded pusher (40), from the insertion position, in the head distal part of the longitudinal slots (24), to the implanted position, in the head proximal part of the longitudinal slots (24), reaching said housing (23) to become there implanted reducing the spinal deformity;
• said threaded elongate member (22) is at least partially removable once corrective rod (30) is implanted,
**characterized in that**
• said threaded elongated member (22) and the receiving part (21) are integrated into the same element;
• said threaded elongated member (22) is cylindrical, and is threaded on its inner surface;
• said slots (24) extend from the threaded elongated member (22) proximal end to the threaded elongated member (22) distal end, completely dividing the threaded elongated member (22) into two halves;
• said threaded pusher (40) is inserted and threaded into said threaded elongated member, and comprises a driving head (42) and at least an implantable portion (43);
• threads (41) of the threaded pusher (40) implantable portion (43) have a zip configuration with protrusions (46), comprising at least a thread distal portion protruding relative to a thread more proximal portion, said zip configuration being complementary with the threads (25) of the threaded elongated member (22).

2. System according claim 1 wherein the threaded elongated member (22) has a weakening zone (26), which facilitates removing a non-implantable portion (27) of the threaded elongated member (22) by breakage or cutting, defining the remaining portion an implantable portion (28) which surrounds said housing (23).

3. System according any preceding claim wherein the threaded pusher (40) has at least an implantable portion (43), able to retain the rod (30) on the implanted position, threaded on the threaded elongated member (22).

4. System according to claim 2 wherein the threaded pusher (40) has a non-implantable portion (44), connected to the implantable portion (43) through a weakening zone (45), which facilitates the removing of the non-implantable portion (44) by breakage or cutting.

5. System according to claim 3, wherein said zip configuration is provided only on the implantable portion (43) of the threaded pusher (40).

6. System according to claim 1, wherein said head (12) is pivotally connected to said receiving part (21) allowing movement in a single plane.

7. System according to claim 1, wherein said head (12) is pivotally connected to said receiving part (21) allowing movement in any direction.

8. System according to claim 1, wherein said head (12) is connected to said receiving part (21) preventing its movement.
